# EUROPEAN PATENT APPLICATION

(11) **EP 1 367 066 A2**
(43) Date of publication of application: **03.12.2003**
(21) Application number: 03253099.0
(22) Date of filing: 17.05.2003
(51) Int. Cl.: C07K 14/78, C09H 3/00, A61K 35/60, A61K 47/48

(54) **Succinylated fish gelatin**

(30) Priority: 21.05.2002 GB 0211626
(71) Applicant: CRODA INTERNATIONAL plc, Goole North Humberside DN14 9AA (GB)
(72) Inventor: Jones, Roger, Trevor, Cuddington Cheshire CW8 2EE (GB)
(74) Representative: Coates, Ian Harold

(57) **Abstract**

A process is described for preparing a gelatin composition suitable for use in the preparation of a blood plasma extender, which process comprises:
(a) pre-conditioning, under alkaline conditions, a gelatin-producing raw material;
(b) preparing, from the pre-conditioned raw material product of step (a), a gelatin starting material having a pl in the range of from 4.5 to 6; and
(c) derivatising the gelatin starting material product of step (b), whereby there is produced derivatised gelatin having a pl in the range of from 4 to 5.

The derivatised gelatin thereby produced preferably has a pl in the range of from 4 to 4.8 and a degree of derivatisation in the range of 50-60%. A succinylated gelatin thereby produced is particularly suitable for use in the preparation of a blood plasma expansion products.

## Description

The present invention relates to the manufacture of gelatin derivatives suitable for use as a blood plasma expander, preferably using alkaline processed fish-derived gelatins as the primary raw material. In particular, the invention relates to the production of succinylated gelatin derived from fish skins.

Gelatin is a protein derived from the natural protein, collagen, which is found in animal species. The collagen of bone and skin is the primary structural protein in the animal kingdom, and this is the material source used for gelatin manufacture.

The use of modified gelatins as blood plasma expanders, extenders or substitutes, for the clinical treatment of patients suffering from shock or other conditions requiring an increase in circulating blood volume, is an established and important application. Such modified gelatins, in the form of sterile, pyrogen-free, dilute isotonic solutions, are able to provide a normal intravascular oncotic pressure, which can persist for several hours following infusion.

Typically, such modified gelatins are employed as 3-5% w/v solutions. 'Conventional' (*ie* un-modified) gelatins will gel when solutions are cooled below their setting points (typically 24°C-32°C, for a 4% solution) and, since the blood plasma expanders are required to remain liquid under all likely storage and usage conditions, the gelatin needs to be 'modified' (*ie* derivatised) to reduce its gelling ability.

Gelatin is available in various grades, having differing average molecular weights. Commercially, gelatins tend to be graded in terms of their gel strength (Bloom value) under standard test conditions, although viscosity is generally also an important parameter, which is more directly related to average molecular weight. Gelatins are commercially available with Bloom gel strengths in the approximate range 50-320g and viscosities (tested on 6.67% solution at 60°C) in the approximate range 2-8 mPas. Average molecular weight values are not normally cited, since there is no universally accepted test procedure for gelatin, and the values for average molecular weights can vary dependent on the test method and procedure used. However, based on the size exclusion hplc method described further hereinafter, the above-mentioned gelatins typically have weight average molecular weights (Mw) in the range 70,000-250,000 Daltons and number average molecular weights (Mn) of 30,000-100,000 Daltons.

Non-gelling gelatins (that is, gelatins with zero Bloom) are also offered commercially, and these are normally produced by using proteolytic enzymes deliberately to hydrolyse the gelatins down to weight average molecular weights of the order 8,000-30,000 Daltons. However, solutions of these hydrolysed gelatins may still gel at temperatures below 10°C (the temperature of the Bloom gel test).

Development of gelatin-based blood plasma volume extenders dates back to the second world war and was further promoted during the Korean war, due to the need to supplement blood supplies for combat casualties. It was soon recognised that hydrolysing gelatin, to the point that 3-6% solutions no longer gelled at room temperature, created other problems: low molecular weight gelatin molecules did not remain in the circulation system long enough to have any beneficial effect.

In an attempt to overcome this limitation, various chemical modifications of the gelatin, or hydrolysed gelatin, were explored. 'Oxypolygelatin' uses glyoxal as a crosslinking agent to build up the molecular weight of hydrolysed gelatin, without increasing the tendency for gelation. This was followed by hydrogen peroxide treatment, claimed to increase the number of ionisable groups on the modified gelatin, at near-neutral pH. The use of an alternative, di-isocyanate crosslinking agent was also explored, which resulted in the commercial product 'Haemacell'.

US patent specification no. 2 827 419 describes reacting gelatin (preferably of number average molecular weight 15,000-36,000) with succinic, citraconic, itaconic, aconitic or maleic anhydride, or succinyl or fumaryl chloride. As a consequence of acylation, these acylated gelatins show lowered iso-ionic points, which results in improved colloid osmotic function, making them more suitable for use as blood extenders.

British patent specification no. 1 475 364 also describes the use of acylated gelatin, particularly succinylated gelatin, as a blood plasma substitute. Here, the problem of solutions gelling at low temperature was overcome by partially hydrolysing the gelatin starting material under alkaline conditions, before succinylation, such that partial racemisation of the L-amino acid residues to the D-form occurs, leading to a reduction in gel melting point, to close to 0°C. The partially hydrolysed gelatin was specified to have a number average molecular weight in the range 20,000-30,000.

However, a significant disadvantage of the hydrolysis step proposed in this disclosure is that it results in the production of ammonia, due to the de-amidation of glutamine and asparagine to form their respective D-acids. Succinic anhydride reacts with amino groups on the gelatin molecule but will also react with other primary amino groups, such as that of ammonia. Conversion of acid-processed gelatin to alkaline-processed gelatin results in release of approximately 0.3 millimoles ammonia per gram of gelatin (see, for example, The Macromolecular Chemistry of Gelatin, Arthur Veis, p103, Academic Press, 1964). This compares with approximately 0.42 millimoles amino groups per gram for the gelatin itself.

This means that either an excess of succinic anhydride needs to be added to react with both the gelatin amino groups and free ammonia, or the ammonia must first be removed by ion exchange, ultrafiltration or other appropriate technique. The first option results in a significant amount of by-product ammonium succinate, which is undesirable. The latter approach has practical limitations, due to potential yield loss and also because it is desirable to avoid any steps, following alkaline hydrolysis, which could possibly introduce endotoxins into the gelatin.

The present invention is concerned with specific improvements to the production of acylated, especially succinylated, gelatins for use in blood plasma replacers/extenders, particularly relating to the type of gelatin starting material to be used.

Commercially, the traditional raw materials for gelatin manufacture have been bones and hides/skins from bovines and porcines. Limited quantities of ovine raw material have also been used. There are essentially two different processes for converting the collagen in these raw materials into gelatin. These are the 'acid process', in which the collagen is denatured and partially hydrolysed simultaneously, by extracting into hot water at acid pH, and the 'alkaline process', in which the collagen is 'conditioned' with alkali (such as lime) prior to extraction in hot water at more neutral pH. Gelatins produced by the acid process are termed 'Type A gelatins' and those processed by the alkaline route are termed 'Type B gelatins'.

Not all raw materials are suitable for alkaline processing. Compared with bovine hide, pigskin contains a high fat content and is exclusively processed by the acid route. This is because fat separates on the surface of the gelatin solution during acid extraction, and can be collected and sold as a valuable by-product. In contrast, the presence of fat in pigskin not only reduces the ability for penetration of alkali into the skin, but also produces soaps, which adversely affect the clarity of the finished gelatin.

Another important property of gelatin is its iso-ionic pH in solution. The iso-ionic pH of a gelatin is the pH of a gelatin solution in which there are no other ionic species present (such as when a gelatin solution has been treated with ion-exchange resin). For de-ionised gelatin solutions, iso-ionic points and iso-ionic points are essentially the same. The iso-ionic point is the pH at which the gelatin has a net zero charge and thereby shows no net migration on application of an electric field.

Type A gelatins possess higher iso-ionic points than Type B gelatins. The lower iso-ionic points of Type B gelatins are due to the conversion of glutamine and asparagine residues to their acid forms under alkaline processing conditions. Typically, acid pigskin gelatins have iso-ionic points in the range pH 7.0-9.5. Acid processed bone and hide gelatins tend to have slightly lower iso-ionic points of pH 6.0-8.0. Alkaline processed gelatins typically have iso-ionic points of pH 4.7-5.2.

Potentially, collagens from non-mammalian sources such as the skins of fish, amphibians, reptilians and avians could also provide raw materials for gelatin manufacture, but these have largely been ignored. Fish glues, however, have been produced for many years, and their reduced gelling properties (related to their lower content of the imino acids proline and hydroxyproline) have been used to advantage. In 1960, Norland Products began to manufacture technical fish gelatin for the electronics industry from cold-water fish skins. This was an acid processed gelatin, offered as a 45% solution and preserved with methyl- and propyl-parabens, under the trade name Hi Pure Liquid Gelatin.

The recent commercial interest in fish gelatin, as an alternative to traditional mammalian gelatin, for many food and pharmaceutical applications has been largely fuelled by concerns about TSEs (transmissible spongiform encephalopathies), particularly BSE (bovine spongiform encephalopathy), since the time the latter was first identified in the UK in 1986. Additionally, fish gelatin, provided it is produced from specific fish species, can be considered kosher, and there is much interest in the use of this type of gelatin in kosher products.

Accordingly, there is a need for blood plasma extension products derived from non-mammalian, eg fish, sources that meet the conflicting requirements of: (a) maintaining fluidity by reducing the product's gel melting point; and (b) maintaining an effective oncotic concentration of the product in the circulating blood (achievable by maintaining or increasing its molecular weight). We have now surprisingly found that these criteria can be met by using fish raw material; pre-conditioning this material under specific conditions; and selecting a specific iso-ionic point (pl) range for the gelatin starting material produced from the pre-conditioned raw material.

Therefore, the present invention provides a process for preparing a gelatin composition suitable for use in the preparation of a blood plasma extender, which process comprises:
(a) pre-conditioning, under alkaline conditions, a gelatin-producing raw material;
(b) preparing, from the pre-conditioned raw material product of step (a), a gelatin starting material having a pl in the range of from 4.5 to 6; and
(c) derivatising the gelatin starting material product of step (b), whereby there is produced derivatised gelatin having a pl in the range of from 4 to 5.

Preferably, the gelatin raw material for step (a) comprises that derived from fish, especially fish skins. It is particularly preferred that frozen or dried skins are used, especially frozen skins, whereby they have been well-preserved. More preferably, the skins are substantially free of other fish offal, such as bones, flesh, heads, and innards. Still more preferred is when the skins are substantially free from the skins or other offal of aquatic mammals, including whales and dolphins. The skins may be in de-scaled form.

Preferably, the fish skins are derived from fish that have evolved in warm water, such as certain tuna species, tilapia and Nile perch. Such warm water species tend to exhibit collagen having the preferred amount of proline and hydroxy-proline residues. Accordingly, the invention further provides a process as described above, wherein the fish skins have a collagen content comprising, per 1000 amino acid residues, more than 110 proline residues and/or more than 60 hydroxyproline residues. In cases where the collagen has an amino acid composition outside these criteria, then step (a) is preferably undertaken at a reduced temperature in the range of from about 15 to about 5°C.

Step (a) is preferably carried out by treating the fish skins with saturated lime solution. Thus, in step (a), lime slurry may be prepared to a concentration range such as from 0.6-8.0% by weight, such as 2 to 8% w/w and preferably 1-2%, although may be 4-6%, especially during the early stages of liming, although these quantities could be reduced, if preferred, for the later stages of liming. Step (a) is undertaken to condition the fish skins in the alkaline (eg saturated lime) solution, such that the amide residues are essentially de-amidated. Preferably, the saturated lime solution and the fish skins are kept in admixture in step (a) for a period of at least 5 days, such as in the range of from 5 to 150 days, such as 12 to 100 days, *eg* 12 to 70, including 12 to 15 days.

The lime liquors are preferably replaced at intervals throughout the liming process and the fish skins should be agitated and preferably aerated at intervals. During the early stages of liming the amide residues in the collagen become de-amidated, thereby releasing ammonia; various non-collagenous proteins and other impurities may be solubilised and removed. The lime treatment allows the collagen to become conditioned, such that extraction of gelatin of high quality can be achieved in subsequent processing.

The alkaline conditioning of the fish skins in step (a) is not restricted to the use of excess calcium hydroxide and may be carried out using various other sources of alkaline materials that allow the production of Type B gelatin. Alkaline materials that could be used instead of or as well as lime include, for example, the oxides and hydroxides of sodium, potassium, lithium, calcium, magnesium and the various mixed salts that incorporate such components. Thus, solutions of sodium hydroxide of suitable concentration, such as 0.1-1 %, *eg* 0.2%, may be used over appropriate time periods, such as 0 hours to 5 days, *eg* about 24 hours. When, for example, caustic soda is used in step (a) instead of lime, 0.6-2% sodium hydroxide solution is preferably used and the mixture with the skins allowed to stand for 1 to 20 days, *eg* about 4 days.

Preferably, the liming or other alkaline treatment is carried out so as to provide a mixture of the fish skins and alkali having a pH of at least 12 and preferably greater than 13.

More preferably, step (a) is preceded and/or immediately followed by washing steps. Furthermore, a preparatory alkalination step, to pre-condition the skins, without causing substantial de-amidation, may precede liming or other alkali treatment. Other additives, such as a swelling suppressant, eg sodium sulphate, may be used, if preferred, to suppress excessive swelling and possible subsequent loss of gelatin yield.

Most preferably, the gelatin starting material is prepared in step (b) by acid extraction, as described in our co-pending British patent application no. 112 331 (the contents of which are incorporated herein by reference). Accordingly, the pre-conditioned raw material is acidified, which may be effected by using a dilute mineral acid, such as dilute sulphuric acid, to enable acid pH, preferably a pH in the range of from 1 to 7, *eg* 2 to 5, such as about 3. Acidification is most preferably undertaken in the absence of added organic acid(s) and/or in the absence of added sulphurous (SO₂) moieties. More preferably, the raw material is soaked at the given pH for up to 24 hours or so.

In order to prevent deterioration of physical properties, such as Bloom and viscosity, of the extracted gelatin, the extraction is carried out at the lowest temperature possible, typically 30-50°C, although elevation to about 60-70°C or higher is practicable, particularly towards the end of the extraction, to help extract all the available gelatin. Heating may be carried out at the given pH, preferably about 3 to 5, for up to 24 hours or so, such as for about 1-2 hours at 55-60 °C. It may not be necessary to add further mineral acid after the acidification step (b).

The extraction may be followed by one or more of the standard separation and/or purification techniques known in the art, including filtration, ion exchange, concentration, sterilisation, drying and the like. Suitably, this includes an ion-exchange reaction using, for example, both anionic and cationic exchange resins to reduce the salts content of extracted liquors. The use of ion-exchange resins in gelatin manufacture is described by P Caimi in Imaging Science Journal 45 136-138 (1997). Resins can be chosen from any suitable for the purpose, including those manufactured by Rescindion or Rohm & Haas.

In steps (a) and (b) of the process of this invention, the fish skins are therefore treated sequentially, preferably in the following stages: defrost the skins, optionally soak in alkali to pre-condition, wash, add lime, transfer to process vats (lime pits) and lime for 10 - 100 days, suitably 12-15 days, transfer to washers, wash, add sulphuric acid to pH about 2.5-4, transfer to extractors; extract at 40-60°C, filter, de-ionise, evaporate, dry, grind and store.

Alternatively, the gelatin starting material is prepared in step (b) by alkaline extraction, as described in US patent specification no. 5 484 888, which describes an alternative alkali process for producing warm water fish skin gelatin.

We have found that the problems of ammonia in the gelatin starting material can be avoided by the use of exclusively alkaline processed gelatin. In these alkali-based processes, the gelatin thereby produced is of low iso-ionic pH of around 5 because amide-group (-CONH₂)-containing amino acids have been converted to acid groups (-COOH). Preferably, the gelatin starting material should have an iso-ionic point of less than pH 5.6, and more preferably it should have an iso-ionic point of 4.7-5.2, especially about 5.0-5.1

The derivatisation step (c) is therefore preferably carried out on gelatin starting material that is substantially free from ammonia. By "substantially free" in this context is meant that the maximum residual ammonia in the starting gelatin is less than 0.02% w/w and preferably less than 0.01% w/w.

For step (c), it has been found that both gelling (warm water fish derived) and non-gelling (cold-water fish derived) gelatins are suitable as the starting gelatins. Each type offers certain advantages and disadvantages. Gelling fish gelatin has an amino acid composition closer to that of mammalian gelatin, when compared with non-gelling fish gelatin, and consequently provides essential similarity to gelatins already used for the production of blood plasma extenders. The derivatisation process results in derivatised gelatins that are non-gelling. On the other hand, non-gelling fish gelatins provide the opportunity for achieving higher average molecular weights for the derivatised gelatin without a 4% solution gelling at zero degrees Celsius. This could potentially increase the retention time in the circulation system.

A base is preferably added, prior to derivatisation, at high pH in the range of from 13 to 14, such as about 14, preferably at elevated temperature, in order to hydrolyse the gelatin, thereby reducing its molecular weight to within a range suitable for use as a blood plasma extender.

Product based on gelling fish gelatin should have a Mw of 30,000-60,000 daltons (and more preferably 40,000-50,000 daltons) and an Mn of 20,000-30,000 daltons. Product based on non-gelling, cold water fish gelatin can have a wider molecular weight range of Mw 30,000-70,000 and Mn 20,000-40,000.

These average molecular weight values are based on a size exclusion hplc procedure. Since there is no universally-accepted test method and conditions for determining average molecular weights of gelatins and different methods can give different values, it is necessary to specify certain details of the test conditions used, in relation to the stated minimum average molecular weights. These are:
Size exclusion column: TSK G4000 SWxl (30cm x 7.8 mm internal diameter)
Pump: Hewlett Packard HP1100 series isocratic pump (G1310A)
Injector: Hewlett Packard HP1100 series autosampler (G1313A)
Thermostat: Hewlett Packard HP1100 series thermostatted column compartment (G1316A)
Detector: Hewlett Packard HP1100 series variable wavelength detector (G1314A)
Control: Hewlett Packard HP1100 series Chemstation software (G2175A)
Integration: Polymer Laboratories Caliber GPC software
Eluent: 0.05M KH₂PO₄, 0.05M K₂HPO₄.3H₂O and 0.1M NaCI adjusted to pH 7.0
Temperature: 25°C
Detector wavelength: 220nm
Calibration molecular weight standards: A limed bone gelatin of high Bloom (typically 240-280g) is used as the reference standard, from which the positions of the alpha, beta, gamma and other components, of established molecular weights, can be identified. These values are used to construct a straight line calibration.

The derivatisation step (c) is preferably carried out by standard methods known in the art by reaction with a suitable reactive compound capable of reacting with lysine and/or amino end-groups of the gelatin starting material. Suitable reactive compounds include acylating compounds, such as acid anhydrides, acid halides (such as acid chlorides) and halohydrins (such as chlorhydrins). Preferred are the anhydrides, such as succinic, maleic, phthalic and others including those described in US patent specification no. 2 827 419. A particularly preferred reactive compound is succinic anhydride and, accordingly, a particularly derivatisation step comprises succinylation.

Succinylation may be carried out by any method known to those skilled in the art, such as is described in US patent specification no. 2 827 419 or British patent specification no. 1 475 364. Preferably, succinic anhydride is reacted, more preferably at elevated temperature (such as 60-70°C), with the gelatin starting material at a pH preferably maintained at about 9-9.5 by the use of a base, such as sodium hydroxide. Other derivatisation steps may be carried out in an analogous manner or by other methods known to those skilled in the art.

The succinylated gelatin thereby produced is pH-adjusted to a pH preferably in the range of from about neutral, such as about 6.8 to 7.2. The product preferably has a pl in the range of from 4 to 4.8, especially about 4.3 to 4.4. Suitably, the product will exhibit a degree of derivatisation, *eg* succinylation, sufficient to prevent gelation in use. A suitable degree of derivatisation comprises, for example, in the range of 50-60%, being the percentage of amino groups in the gelatin starting material available for reaction with the reactive compound, *eg* succinic anhydride, that have in fact so reacted.

The present invention is therefore especially concerned with the use of alkaline processed fish gelatin for the production of succinylated gelatin. This not only offers the practical advantages identified for alkaline processed gelatin but also addresses potential concerns relating to TSE infectivity. This invention therefore further provides:
(a) a derivatised, especially succinylated, gelatin prepared according to a process of this invention;
(b) the use of alkaline-processed gelatins, especially those derived from fish, in the preparation of a derivative, especially a succinylate, thereof for use in blood plasma expansion products;
(c) a blood plasma expander composition comprising a derivatised gelatin according to (a), especially a gelofusion composition comprising a solution of (a);
(d) the use of a derivatised gelatin according to (a) in the preparation of a blood plasma expansion product; and
(e) a method of treating a patient in need thereof, which method comprises administration to the patient of a derivatised gelatin according to (a) or a blood plasma expander composition according to (c).

The suitability of alkaline processed fish gelatins for the production of derivatised gelatin, especially succinylated gelatin for blood plasma extenders, is illustrated by the following Examples.

### Description 1 - Starting Material Preparation of Low pl Fish Gelatin using the Liming Process

Tilapia skins (2156g) were added to 0.2% sodium hydroxide solution (8064g), mixed and allowed to stand for 24 hours. The sodium hydroxide solution was drained off and the skins rinsed with water (8000g). The skins (3991g) were then added to a solution of lime (1.2%, 8100g and left for 12 days. After 12 days the lime solution was removed and the skins washed with water (8000g).

The skins (3764g) were then added to water (5000g), and the pH adjusted and maintained between 2.5-4.0 with the addition of sulphuric acid (77%) over a period of 6 hours. The skins were then allowed to soak at this pH for a further 17 hours. The acid conditioned skins were then heated to 60°C for 2 hours, whilst maintaining the pH at 4.0 with sulphuric acid (77%). After 2 hours the slurry was coarsely filtered to remove the waste skin residue and the resultant extraction filtrate was filtered through a Dicalite 4258 coated W2 filter pad. The filtrate (8812g, rf=6.4, pH=4.06) was heated to 55°C and treated with MB6113 mixed ion-exchange resin (950.0g) until the pH was stable (pH=5.65, conductivity=34µS). The filtrate was then filtered through a BECO-KD5G filter pad to remove the ion-exchange resin, followed by filtration through an XE200H filter pad to clarify. The filtrate (7913g, rf=5.5) was evaporated to rf = 8.8 (5369g) and poured into a tray and allowed to gel at 4°C, noodled, dried and ground to give Tilapia fish gelatin (446.2g).

| Analysis | |
|---|---|
| **Test** | |
| Moisture (%) | 15.6 |
| Ash (%) | <0.1 |
| Nitrogen (%) | 15.6 |
| Hydroxyproline (%) | 10.2 |
| Bloom @ 6²/₃ % (g) | 240 |
| pH ( 1 %, 25°C ) | 5.8 |
| Iso-ionic pH | 5.7 |
| Viscosity (@ 6²/₃ %, 60°C) (mPas) | 4.20 |

### Description 2 - Starting Material Preparation of Low pl Gelatin using the Caustic Process

Tilapia skins (4341g) were added to sodium hydroxide solution (1.2%; 19000g), mixed and allowed to stand for 24 hours. The sodium hydroxide solution was then drained off and the skins rinsed with water (19000g). The skins (11297g) were then added to fresh sodium hydroxide (1.2%; 12600g), mixed and allowed to stand for 4 days. The sodium hydroxide solution was then drained off and the skins rinsed with water (12600g).

The skins (15224g) were added to water (10000g), and the pH adjusted and maintained between 2.5-4.0 using sulphuric acid (77%) over a period of 6 hours then allowed to soak at this pH for a further 18 hours. The acid conditioned skins were heated to 55°C for 1¹/₂ hours, whilst maintaining the pH at 4.0 with sodium hydroxide solution (25%). After 1¹/₂ hours the liquor was coarsely filtered to remove the waste skin residue and the resultant extraction filtrate was filtered through a Dicalite 4258 coated W2 filter pad. The filtrate (pH=4.0) was heated to 55°C and treated with MB6113 mixed ion-exchange resin (3678g) until the pH was stable (pH=5.6, conductivity 7.0µS). The filtrate was then filtered through a BECO-KD5G filter pad to remove the ion-exchange resin, followed by filtration through an XE200H filter pad to clarify. The filtrate was evaporated to 10.2 % (9322g) and poured into a tray and allowed to gel at 4°C, noodled, air dried and ground to give low pl Tilapia fish gelatin (906.8g).

| Analysis | |
|---|---|
| **Test** | **Result** |
| Moisture (%) | 16.5 |
| Ash (%) | <0.1 |
| Nitrogen (%) | 15.3 |
| Hydroxyproline (%) | 9.8 |
| Bloom @ 6²/₃ % (g) | 180 |
| pH (1%, 25°C) | 5.7 |
| Iso-ionic pH | 5.7 |
| Viscosity (@ 6²/₃ %, 60°C) (mPas) | 4.08 |

### Description 3 - Alternative Production of Type B Gelatin Starting Material

A sample of frozen tilapia skins was allowed to thaw out in water containing sodium hypochlorite.

The skins were given a wash in water (1 hour) to remove some of the fat present. A 2191g sample of the drained tilapia skins (42 pieces) was placed in a 10-litre container. Water was added to 8 litres and then 100g lime was added.

The lime treatment was carried out in the laboratory at ambient temperature (19-25°C). The skins were stirred occasionally. Lime changes were carried out after 5 days and after 34 days by draining off the lime liquor and replacing with water and 100g lime. The skins remained tough throughout the liming period.

After 42 days, the skins were divided into two aliquots of 2100g (This is almost double the weight before liming). One portion (B) was returned to the lime liquor to continue liming. The other portion (A) was extracted.

### Extraction A: Extraction of Tilapia skins after 42 days in lime

The skins were washed in running water over 30 minutes before transferring to a 5 litre polypropylene beaker and covering with water. Sulphuric acid was added over 6 hours to lower the pH to a liquor pH of around pH 3. After 3 hours the liquor pH was 5.0, and the liquor was drained off and replaced with de-ionised water. After a further 2 hours the water was again drained off and replaced with de-ionised water before leaving overnight at pH 3.9. About 18ml concentrated sulphuric acid was used. At this stage, about 20% of the area of the skins was transparent; some skins were swollen and very soft, whereas others were white opaque and quite tough.

The gelatin was extracted on heating to 40°C in a water bath over 2-4 hours. The liquor was filtered, de-ionised and the pH adjusted to pH ca 5.5 with sodium hydroxide before being allowed to dry on trays at room temperature. Some of the skins were not extracted within 4 hours and were dried as unextracted residue.

| Gelatin yield | | | |
|---|---|---|---|
| A1 | filtered liquor | 2800ml at 3.3% (92.4g) | 76.3g dry |
| A2 | unfiltered liquor | 900ml at 7.0% (63g) | 49.6g |
| A3 | unextracted residue | | 71.8g |
| Weight of wet tilapia skins used = | | 1095.5g | |
| Yield of dry gelatin = | | 125.9g | 11.5% |
| Total weight recovered = | | 197.7g | 18.0% |

### Extraction B: Extraction of Tilapia skins after 105 days in lime

The skins were washed in running water over 30 minutes to remove excess lime. The weight after draining was 2733g. The skins were transferred to a 5 litre polypropylene beaker and covered with water. Sulphuric acid (13ml) was added over 3 hours to lower the pH to a liquor pH of around pH 5.5. The liquor was drained off and the skins re-weighed (2075g). De-ionised water was added and a further 5ml sulphuric acid added over 2 hours to give a liquor pH of around 3.0. On leaving overnight, the skins had swollen and the liquor pH was 4.1. Most of the skins were soft with some transparency, but about 10-20% were white opaque and less soft.

The gelatin was extracted on heating to 30-40°C in a water bath over 2-4 hours. The liquor was filtered, de-ionised and allowed to dry on trays at room temperature. Some of the skins were not extracted within 4 hours and were dried as unextracted residue.

| Gelatin yield | | | |
|---|---|---|---|
| B1 | filtered liquor | 3820g at 3.7% (141.3g) | 133.4g |
| dry | | | |
| B2 | unextracted residue | | 42.6g |
| Weight of wet tilapia skins used = | | 1095.5g | |
| Yield of dry gelatin = | | 133.4g | 12.2% |
| Total weight recovered = | | 176.0g | 16.1% |

### Dried gelatin test results

| **Test/Sample** | **A1** | **B1** |
|---|---|---|
| Bloom g | 288 | 246 |
| Viscosity mPas (6 2/3% 60°C) | 3.67 | 5.69 |
| pH | 5.24 | 4.95 |
| Conductivity uS (6 2/3% @25°C) | 220 | 90 |
| Colour (absorbance of 6 2/3% solution at 420nm) | 0.252 | 0.302 |
| Clarity (absorbance of 6 2/3% solution at 650nm) | 0.098 | 0.098 |
| Ash % | <0.1 | <0.1 |
| Iso-ionic pH | 5.13 | 5.11 |
| Moisture % | 15.2 | 15.6 |

Tilapia skins can therefore be used to produce limed gelatin in good yield, and with good chemical and physical properties.

### Example 1

Alkaline processed fish gelatin (according to Description 1) (500g dry weight) was added to water to make up a 15% solution. This was heated to 70+1°C and stirred to make a homogenous solution. Sodium hydroxide solution (25%, 67.5g) (pH 14) was then added to the gelatin solution and the temperature was maintained at 70±1°C for 13 minutes. The pH of the solution was then adjusted to 9.0 - 9.5 using concentrated hydrochloric acid.

The reaction mixture was then cooled to 63±1°C and succinic anhydride (15g) was added in six equal aliquots after 0. 12, 24, 36, 48 and 60 minutes. The temperature was maintained at 63±1°C during this addition and the pH was also maintained between 9 - 9.5 using sodium hydroxide solution (25%).

The reaction mixture was then kept at 63+1°C for a further 35 minutes. The pH of the resultant solution was lowered to 6.8 - 7.2 using hydrochloric acid (28%) before it was spray dried on a pilot Niro production minor spray dryer.

### Analysis of Product of Example 1

| **Test** | **Result** |
|---|---|
| Moisture | 4.7% |
| Ash | 6.4% |
| pH (1 % soln. @ 25°C) | 7.3 |
| Iso-ionic pH | 4.4 |
| Viscosity (4% soln. @ 25°C) (mPas) | 2.25 |
| Hydroxyproline | 10.5% |
| Chloride | 2.5% |
| Sodium | 3.5% |
| Degree of succinylation | 51.9% |
| Heavy metals | <30ppm |
| Arsenic | <1ppm |
| Calcium | 250ppm |
| Polydispersity | 1.84 |
| No. Ave. MWt (Mn) | 26748 |
| Wt. Ave, MWt (Mw) | 49143 |

### Example 2

Alkaline processed fish gelatin (according to Description 2) (468g dry weight) was added to water to make up a 15% solution. This was heated to 70±1°C and stirred to make a homogenous solution. Sodium hydroxide solution (25%, 67.5g) (pH14), was then added to the gelatin solution and the temperature was maintained at 70±1°C for 16 minutes. The pH of the solution was then adjusted to 9.0 - 9.5 using hydrochloric acid (conc.).

The reaction mixture was then cooled to 63+1°C and succinic anhydride (14g) was added in six equal aliquots after 0. 12, 24, 36, 48 and 60 minutes. The temperature was maintained at 63+1°C during this addition and the pH was also maintained between 9 - 9.5 using sodium hydroxide solution (25%). The reaction mixture was then kept at 63±1°C for a further 35 minutes. The pH of the resultant solution was lowered to 6.8 - 7.2 using hydrochloric acid (28%) before it was spray dried on a pilot Niro production minor spray dryer.

### Analysis of Product Example 2

| **Test** | **Result** |
|---|---|
| Moisture | 5.0% |
| Ash | 6.2% |
| pH (1 % soln. @ 25° C) | 7.7 |
| Iso-ionic pH | 4.3 |
| Viscosity (4% soln. @ 25° C) (mPas) | 2.20 |
| Hydroxyproline | 10.9% |
| Chloride | 2.6% |
| Sodium | 3.7% |
| Degree of succinylation | 55.0% |
| Heavy metals | <30ppm |
| Arsenic | <1 ppm |
| Calcium | 251 ppm |
| Polydispersity | 1.77 |
| No. Ave. MWt (Mn) | 26888 |
| Wt. Ave, MWt (Mw) | 47505 |

### Example 3 - Gelofusion Formulation (1)

The succinylated gelatin powder prepared according to Example 1 is dissolved in pyrogen-free water to make a 4% w/v solution, which is then filtered and filled into standard, plastic infusion bags. The filled bags are then sterilized by autoclaving.

### Example 4- Gelofusion Formulation (2)

The procedure of Example 3 is followed, but replacing the succinylated gelatin of Example 1 with that of Example 2, and making a 3% w/v solution.

## Claims

1. A process for preparing a gelatin composition suitable for use in the preparation of a blood plasma extender, which process comprises:
(a) pre-conditioning, under alkaline conditions, a gelatin-producing raw material;
(b) preparing, from the pre-conditioned raw material product of step (a), a gelatin starting material having a pl in the range of from 4.5 to 6; and
(c) derivatising the gelatin starting material product of step (b), whereby there is produced derivatised gelatin having a pl in the range of from 4 to 5.

2. A process according to claim 1, wherein the gelatin raw material for step (a) comprises that derived from fish skins.

3. A process according to claim 1 or claim 2, wherein the gelatin raw material for step (a) comprises fish skins substantially free of other fish offal, including bones, flesh, heads, and innards.

4. A process according to any preceding claim, wherein the gelatin raw material for step (a) comprises fish skins derived from fish that have evolved in warm water, including tuna species, tilapia and Nile perch.

5. A process according to any preceding claim, wherein the gelatin raw material for step (a) comprises fish collagen comprising, per 1000 amino acid residues, more than 110 proline residues and/or more than 60 hydroxyproline residues.

6. A process according to any of claims 1 to 4, wherein step (a) is undertaken at a reduced temperature in the range of from about 15 to about 5°C.

7. A process according to any preceding claim, wherein step (a) is carried out by treating the raw material with an alkaline reagent selected from: the oxides and hydroxides of sodium, potassium, lithium, calcium, magnesium and the various mixed salts that incorporate such components.

8. A process according to any preceding claim, wherein step (a) is carried out by treating the raw material with a saturated calcium hydroxide solution to form a lime slurry having a concentration in the range of from 0.6 to 8.0% by weight.

9. A process according to any preceding claim, wherein, in step (a), the raw material is kept in alkaline conditions for a period of at least 5 days.

10. A process according to any preceding claim, wherein, in step (a), the raw material is kept in alkaline conditions for a period of at least 12 days.

11. A process according to any of claims 1 to 7, wherein step (a) is carried out by treating the raw material with caustic sodium hydroxide to a concentration in the range of from about 0.1 to 2%.

12. A process according to claim 11, wherein, in step (a), the raw material is kept in alkaline conditions for a period in the range of from 1 to 20 days.

13. A process according to any preceding claim, wherein, in step (a), the alkaline conditioning is carried out so as to provide a mixture of the raw material and alkali having a pH of at least 12.

14. A process according to any preceding claim, wherein, in step (b), the gelatin starting material is prepared by acid extraction.

15. A process according to any preceding claim, wherein, in step (b), the gelatin starting material is prepared by extraction using a sulphuric acid.

16. A process according to any preceding claim, wherein, in step (b), the gelatin starting material is prepared by extraction in the absence of added organic acid(s) and/or in the absence of added sulphurous (SO₂) moieties.

17. A process according to any preceding claim, wherein, in step (b), the gelatin starting material is prepared by extraction at 55-60 °C.

18. A process according to any preceding claim, wherein, the gelatin starting material has an iso-ionic pH (pl) of less than pH 5.6.

19. A process according to any preceding claim, wherein, the gelatin starting material has an iso-ionic pH (pl) in the range of from 4.7 to 5.2.

20. A process according to any preceding claim, wherein, the derivatisation step (c) is carried out on gelatin starting material that is substantially free from ammonia.

21. A process according to any preceding claim, wherein, a base is added, prior to derivatisation, at a pH in the range of from 13 to 14.

22. A process according to any preceding claim, wherein the derivatisation step (c) is carried out by reaction with a reactive compound capable of reacting with lysine and/or amino end-groups of the gelatin starting material.

23. A process according to any preceding claim, wherein the derivatisation step (c) is carried out by reaction with a reactive compound selected from: acylating compounds, including acid anhydrides, acid halides and halohydrins

24. A process according to any preceding claim, wherein the derivatisation step (c) comprises succinylation.

25. A process according to any preceding claim, wherein the derivatisation step (c) is followed by pH-adjustment of the derivatised gelatin thereby produced to a pH in the range of from about 6.8 to 7.2.

26. A derivatised gelatin produced by or producible by a process according to any preceding claim.

27. A derivatised gelatin according to claim 26, which has a pl in the range of from 4 to 4.8.

28. A derivatised gelatin according to claim 26 or 27, which has a pl in the range of from 4.3 to 4.4.

29. A derivatised gelatin according to any of claims 26 to 28, which exhibits a degree of derivatisation in the range of 50-60%.

30. A succinylated gelatin according to any of claims 26 to 29.

31. The use of an alkaline-processed gelatin of fish origin in the preparation of a derivative, including a succinylate, thereof for use in blood plasma expansion products.

32. The use of a derivatised gelatin according to any of claims 26 to 30 in the preparation of a blood plasma expansion product.

33. A gelofusion composition comprising a solution of a derivatised gelatin according to any of claims 26 to 30.
